# EUROPEAN PATENT SPECIFICATION

(11) **EP 4 271 299 B1**
(45) Date of publication and mention of the grant of the patent: **05.02.2025**
(21) Application number: 21847541.6
(22) Date of filing: 24.12.2021
(51) Int. Cl.: A61B 18/24, A61B 18/14

(54) **MULTI-SOURCE TISSUE ABLATION SYSTEM FOR THE INTERNAL TREATMENT OF PARENCHYMAL ORGANS, HOLLOW ANATOMICAL CONDUITS OR BLOOD VESSELS**
MEHRQUELLENGEWEBEABLATIONSSYSTEM ZUR INNEREN BEHANDLUNG VON PARENCHYMALEN ORGANEN, HOHLEN ANATOMISCHEN LEITUNGEN ODER BLUTGEFÄSSEN
SYSTÈME D'ABLATION DE TISSU MULTI-SOURCE POUR LE TRAITEMENT INTERNE D'ORGANES PARENCHYMATEUX, DE CONDUITS ANATOMIQUE OU DE VAISSEAUX SANGUINS CREUX

(30) Priority: 29.12.2020 EP 20217723
(43) Date of publication of application: 08.11.2023
(73) Proprietor: GEM S.r.l., 55049 Viareggio (LU) (IT); MYRA Medical Sàrl, 1073 Savigny (CH)
(72) Inventor: BRANCHETTI, Lodovico, Northamptonshire NN18 8GG (GB); DI CECIO, Mario, 25060 Marcheno (Brescia) (IT); PASQUINO, Enrico, 1073 Savigny (CH); DI MODUGNO, Nicola, 70037 Ruvo di Puglia (Bari) (IT)
(74) Representative: Grosfillier, Philippe
(86) International application number: PCT/IB2021/062288
(87) International publication number: WO 2022/144741

(56) References cited:
- WO-A1-2020/227086
- US-A- 5 653 684
- US-A1- 2012 022 512
- US-A1- 2012 143 099
- US-A1- 2016 374 752
- US-A1- 2017 119 470
- US-A1- 2017 215 936
- US-A1- 2018 280 070
- US-A1- 2019 374 276

## Description

### Field of invention

The invention relates to the tissue ablation within parenchymal organs, hollow anatomical conduits or blood vessels. It more precisely relates to the use of different kinds of electromagnetic (EM) waves, namely radiofrequency (RF), microwaves (MW) and laser (LS) .

### Background

During the past two decades, imaging-guided tumor ablation (IGTA) that used either chemical or thermal energy has emerged as one of the most effective loco-regional treatment modalities for small malignant hepatic tumors. The first tumor ablation technique to be introduced to clinical practice was the percutaneous ethanol injection (PEI), which is a chemical ablation of hepatocellular carcinomas (HCCs). In the early 1990s, however, thermal ablation using radiofrequency (RF) was developed and proved its superiority to PEI in terms of better survival and local control of the disease in patients with early-stage nonsurgical HCCs. Thereafter, other types of IGTA techniques such as microwave ablation (MWA), cryoablation, laser ablation, irreversible electroporation and high-intensity focused ultrasound (US) were developed and adopted for the treatment of malignant liver tumors. Among them, radiofrequency ablation (RFA) has been the most widely used method of IGTA for small malignant hepatic tumors, particularly HCC and colorectal cancer liver metastasis (CRLM), due to its safety and effectiveness as well as a reasonably good clinical outcome. Currently, it is unknown whether novel technologies will expand the clinical role of image-guided ablation and improve long-term patient outcomes with respect to RFA.

RFA treatment of stenotic atherosclerotic plaques in arterial blood vessels (coronary or peripheral arteries) has been recently proposed to be investigated besides the conventional mechanical balloon angioplasty or drug eluting stenting or ballooning.

### Radiofrequency (RF)

Regarding RFA for the management of patients with HCCs, previous studies have reported that the overall survival after RFA for early-stage HCC was similar to that of surgical resection. Radiofrequency has been the most studied ablation modality in the treatment of colorectal liver metastases.

The wide range of local tumor progression (LTP) reported for the percutaneous approach (12%-48%), when compared to resection, limited its use to highly selected patients with small, well-positioned tumors or with liver recurrences after hepatectomy. Ablation with sufficient radiographic margins and histologically proven necrosis significantly lower LTP.

RFA uses an alternating electric current oscillating between 200 and 1200 kHz [15]. The radiofrequency electrode acts as a cathode. The ions of targeting tumors adjacent to the electrode tip vibrate rapidly in response to these alternating currents. This vibrating friction energy is transformed into heat, while energy deposition drops exponentially away from the tip. Tissue interaction with the temperature induced by radiofrequency is similar to that of laser. The revised 2017 thyroid RFA guidelines by Korean Society of Thyroid Radiology suggest several standard techniques. For benign thyroid nodules, perithyroidal lidocaine injection is recommended to control pain. The trans-isthmic approach and moving-shot technique are essential for thyroid lesions. This technique is useful to minimize complications and marginal nodule regrowth. Recently, a novel technique, named "vascular ablation technique" has been reported. Two different vascular ablation techniques are suggested: artery-first ablation and marginal venous ablation (venous staining). These techniques have the potential to enhance treatment efficacy and reduce the risk of regrowth. For recurrent thyroid cancers, guidelines recommend careful evaluation of critical structures before ablation, hydro-dissection to reduce the risk of thermal damage to surrounding critical structures, and the moving-shot technique.

Among possible complications, nerve damages are the most serious and feared one during RFA. Particularly, voice change induced by thermal damage of recurrent laryngeal nerve is the most common complication related to nerve damage. To lower the risk of damage, some technical measures have been suggested. Recently, cold 5% dextrose solution injection was introduced to treat nerve damage during ablation. If symptoms of nerve damage occur during RFA, such as voice change, palpitations, Horner syndrome, shoulder movement problems, or paresthesia, ablation should be immediately stopped and a cold 5% dextrose solution is injected directly into the space in which the nerves were located. In most patients, the cold fluids can treat effectively the thermal damage of nerves. Most operators performing thyroid RFA use a thyroid- dedicated straight-type internally cooled electrode with short shaft (7 cm) and small diameter (18-19 gauge). In small recurrent thyroid cancers or parathyroid lesions, guidelines recommend 19-gauge electrode tip (i.e., 3.8 mm or 5 mm active tips). A recently introduced thyroid-dedicated bipolar electrode has been introduced for pregnant women and for patients carrying electrical devices (i.e., pacemaker).

Radiofrequency emission has been successfully tested in-vivo, in animal model, on atherosclerotic plaques induced in peripheral vessels. The effects of RFA on atherosclerotic plaques and the vessels structures has been studied and it can be stated that it doesn't induce any anatomical damages to the vessel tunicae. The action of RFA on the plaque is evident with decellularization and significant reduction of the neovessels in the tunica media and intima. The cell decellularization is mainly in charge of smooth muscle cells (SMC) responsible for the atherosclerotic plaque proliferation and the vessel restenosis after angioplasty. The inventive technology reported in this patent is also based on the application of RFA alone or associated with MW or LS together with stenting of blood vessels. It means that energy emission will be associated during vessel stenting or restenosis.

### Microwave (MW)

Microwave (MW) ablation is gaining consensus in the ablation of liver tumors with the hope of capitalizing on its potential benefits over RF ablation, which were demonstrated mainly in animal studies. These include no need for grounding pads, less susceptibility to the heat sink phenomenon, larger ablation zones, shorter ablation times, and possibly better local tumor control. Previous clinical comparisons of MW ablation to RF ablation suggested an advantage for MW ablation with regard to local tumor control for CLM.

MW ablation proved to be resilient to the heat sink effect compared to RF ablation, offering good control for perivascular tumors. The LTP rates for the small number of CLMs treated are similar between RF ablation and MW ablation. The use of MW ablation can be preferred to RF ablation due to its improved ability to treat perivascular tumors, as well as its ability to achieve ablation in significantly reduced times and with no need for grounding pads.

### Laser (LS)

Laser (an acronym for 'light amplification by stimulated emission of radiation') is a highly coherent, collimated and monochromatic energy that can be precisely delivered into small targets from a primary source or through optical fibers that allows a great variability in length and shape of applicators. Common primary sources are laser diode or neodymium-yttrium aluminum garnet, which produce optical wavelengths of 820 nm or 1064 nm. When laser light hits the target, a local increase of temperature occurs, causing permanent damages such as coagulative necrosis (46-100 °C) and tissue carbonization/vaporization (100-110°C). The grade and rapidity of tissue damage depend on many factors, including the amount of energy released, the application time, the vascularization and the water content of the tissue. Laser ablation in the neck is usually performed under ultrasound (US) guidance, which allows a real-time monitoring of the procedure.

Before ablation, a comprehensive assessment of the target lesion to treat is performed with US or contrast-enhanced US (CEUS). The size and shape of the nodule, along with the spatial relations with adjacent organs needs to be carefully evaluated to avoid partial treatments or injury to surrounding organs. Local anesthesia is generally used, with or without conscious sedation depending on patient anxiety and operator preference. According to the size and shape of the nodule, one or more 21-gauge needles are inserted under real-time US guidance in the deepest portion of each nodule. Up to four needles can be inserted simultaneously. A distance of 1 cm should be ideally maintained between inserted needles. Then, a 300 mm diameter plane-cut quartz optical fiber is inserted and advanced up to the introducer needle tip. The introducer needles are then pulled back to expose the tip of each fiber at least 5 mm in direct contact with the target tissue. Common protocols are based on a mean power of 3-5 W, for a total energy delivery of 1200-1800 J for every illumination. After the first illumination, the fiber(s) can be withdrawn by 1/1.5 cm and a subsequent illumination can be performed (*pull-back* technique) until the whole target has been illuminated. Tissue ablation can be monitored in real time following the enlargement of hyper-echogenicity due to gas formation during treatment. For benign nodules, the aim of the treatment is to achieve a volume reduction of at least >50%, and a variable amount of viable nodular tissue is generally maintained in the periphery of the nodule, also in order to reduce the risk of possible complications. Conversely, for malignant nodules, complete tissue destruction needs to be achieved. Thus, laser ablation for malignant nodules requires a careful assessment of the surrounding structure and an idoneal localization of the nodule, ideally 5 mm far from the thyroid capsule. Hydro dissection can be performed to displace other structures close to the tumor. After the ablation, CEUS can be performed to better identify the actual extent of the ablation area, which can be generally overestimated by the gas formed during ablation; a further ablation can be performed during the same session in case CEUS demonstrate untreated areas. Laser ablation is generally performed as an outpatient procedure that does not require particular medication apart corticosteroids or analgesics in the rare case of post-ablation pain.

According to the state of the art, the most promising oncologic treatments are based on emission of three different energy sources, namely radiofrequency (RF), Microwave (MW) and Laser (LS). It appears clear, from the clinical data on comparative studies involving RF vs. MW or RF vs. LS, that each energy source has limits and advantages.

European patent application EP3355821A1 discloses several devices that each provide one of those three energy sources.

US patent application 2019/374 276 A1 discloses a device that may provide MW and LS but not simultaneously or in a combined manner.

Accordingly, there is a need to provide all three energy sources in a more convenient way.

### General description of the invention

The present invention concerns a tissue ablation system for the internal treatment of parenchymal organs, hollow anatomical conduits or arterial/venous blood vessels; said system comprising an EM wave generator and a needle or catheter with an active distal end. The system according to the invention is characterized by the fact that the generator includes at least two EM wave outputs and is adapted to provide three types of EM waves through said outputs, namely RF, MW and LS; said generator furthermore comprising a processing unit that is programmed, among other things, to emit all three EM waves at the same time and control the interaction among them.

In a preferred embodiment, the system according to the invention provides the three different EM waves separately during a same procedure.

The system according to the invention may deliver RF and MW in alternate switch modality of pulses at millisecond intervals. The system may deliver RF and LS or other EM waves combinations. The combined EM waves can be applied with a needle able to create variable ablation field volumes with cooling and precise local temperature control.

In a preferred embodiment the system comprises a navigation catheter and an active rapid-exchange ablation needle or catheter placed coaxially inside it.

The navigation catheter is conceived as a hub able to bring a rapid-exchange transponder needle precisely into the tumoral lesion to be treated. The active ablation needles/catheters are advanced inside the navigation catheter to reach the lesion to be treated. The operator, through the navigation catheter, can monitor the advancement of the coaxial rapid-exchange needle or catheter controlling the dimension of the tissue ablation volume.

The navigation catheter is placed in position on the skin of the patient to get the right introduction angle. A needle, equipped with a transponder is introduced through the navigation catheter, guided by a dedicated system (computerized tomography or echography), to reach a precise positioning into the tumoral lesion. The transponder needle is then removed and replaced by different rapid-exchange active ablation needles or catheters, with simple or combined energy features (e.g., RF+MW or RF+LS) depending on the organ to be treated or the dimension of the tumor lesion.

The needles or catheters are preferably small and tapered, to reduce the tissues' damages during their progression into the tissues. The needle/catheter dimensions and shape are depending from the organ to be treated, from the type and power of the energy delivered and from the presence or not of a cooling system. For example, needles may have a diameter of 16 G (Gauge) or smaller and the catheters a diameter of 6 Fr (French) or smaller. The above-mentioned needles or catheters can be equipped with a specific flushing system placed at their distal end.

An additional optional feature of the system according to the invention is a mechanical biopsy sampler placed on the tip or replacing the tip that is also acting as electrode. This feature makes it possible to harvest tissue samples for histologic assessments after an ablation procedure.

The use of a needle/catheter is indicated for the following listed organs, but it does not preclude the presence of possible ablation zones in other organs: liver, thyroid, kidney, lung, bladder, brain, hypophysis, pancreas, etc.

The use of catheters is specifically indicated for all tissue ablations in hollow organs such as, for example, brain blood circulatory vessels, heart blood vessels (coronary arteries), arterial or venous blood vessels such as the pulmonary veins (arrythmia treatment), kidney's blood vessels (blood pressure control), respiratory tree, liver biliary ducts, gastro-intestinal tract, bladder, upper or lower ureteral tract conduits or reproductive organ cavities.

At least two associated EM wave types are simultaneously emitted. The generator and its processing unit are designed to control the ablated tissue temperature and impedance during the procedure, depending on the selected energy sources, acting on the cooling pump, placed onboard of generator, that can be based on liquid CO₂, refrigerated air or water.

### Detailed description of the invention

The invention will be better understood in the present chapter, with some non-limiting illustrated examples.

### Brief description of the figures

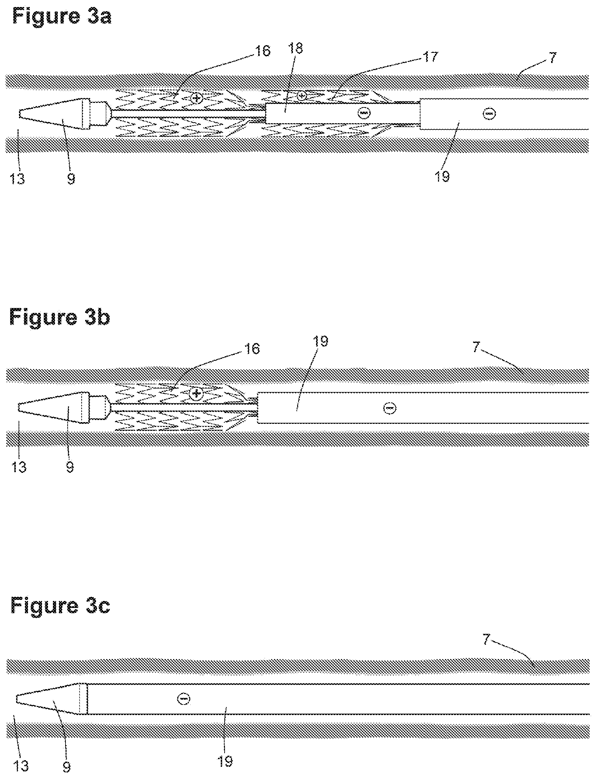

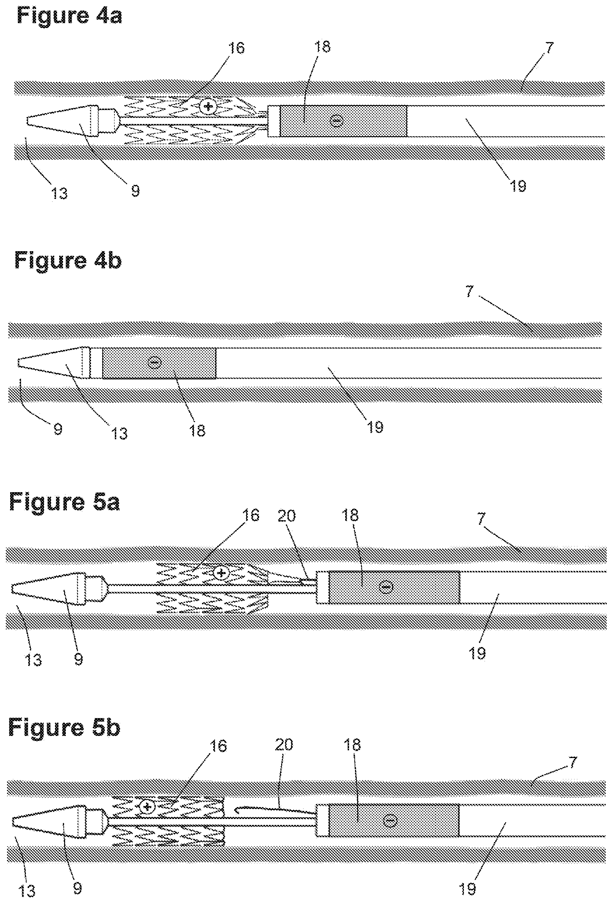

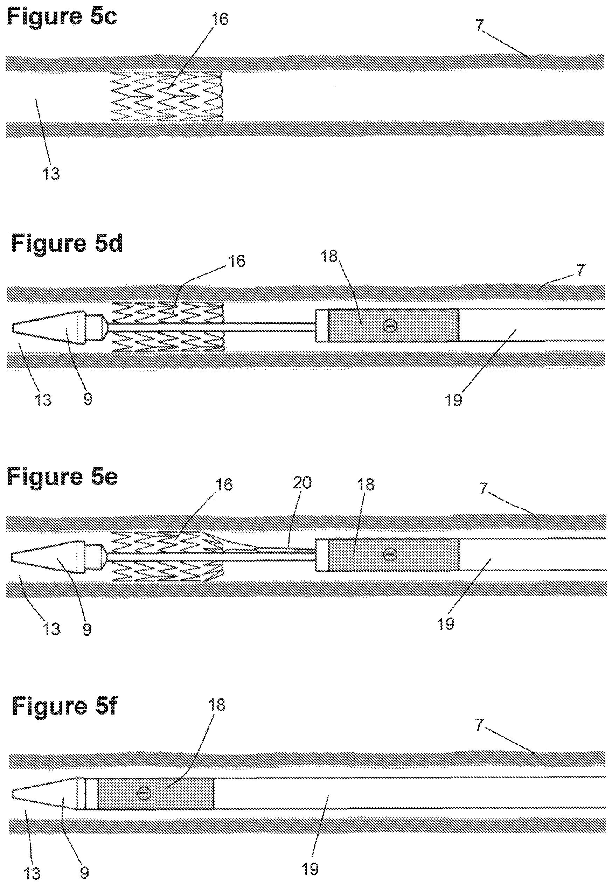

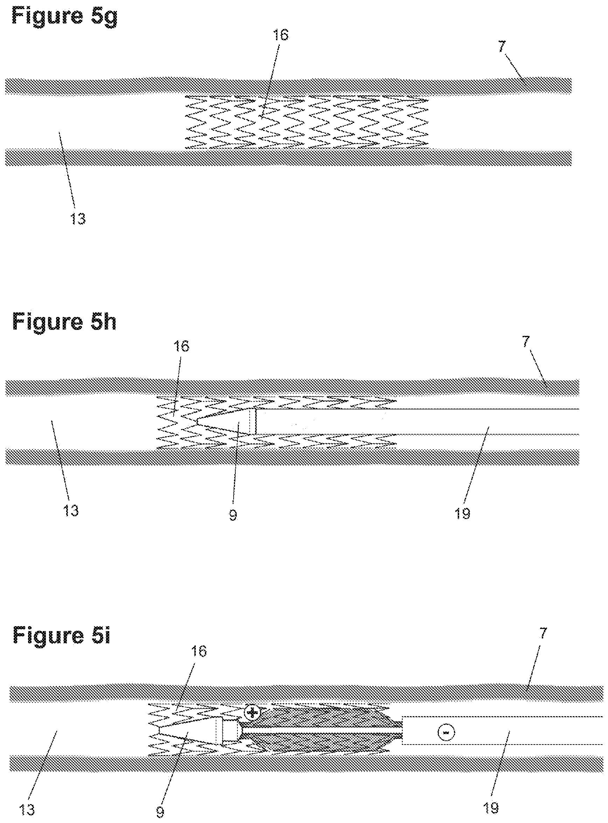

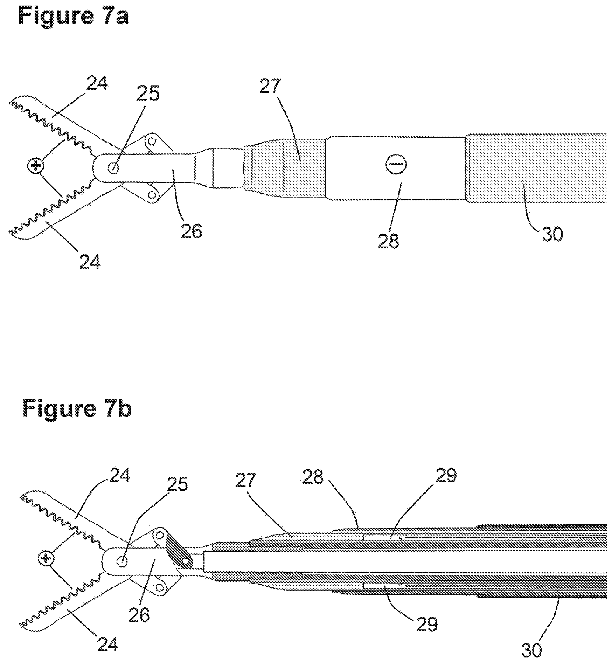

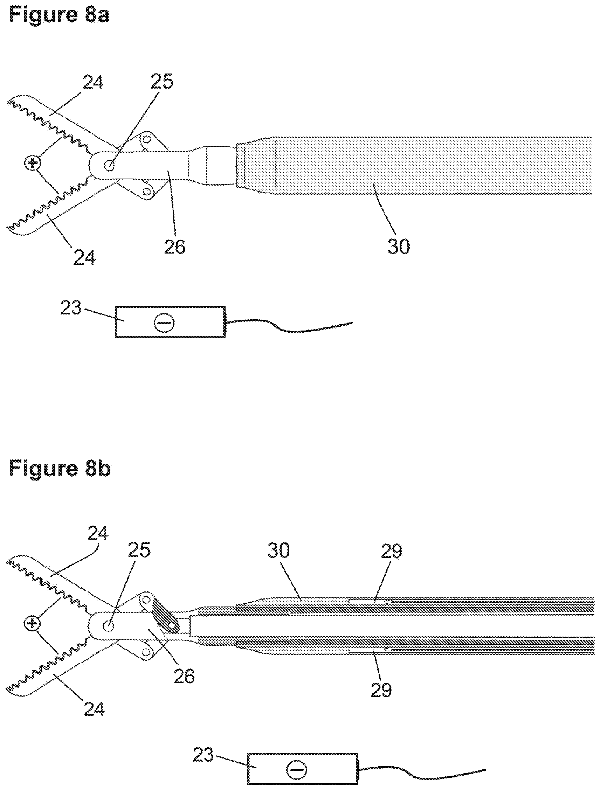

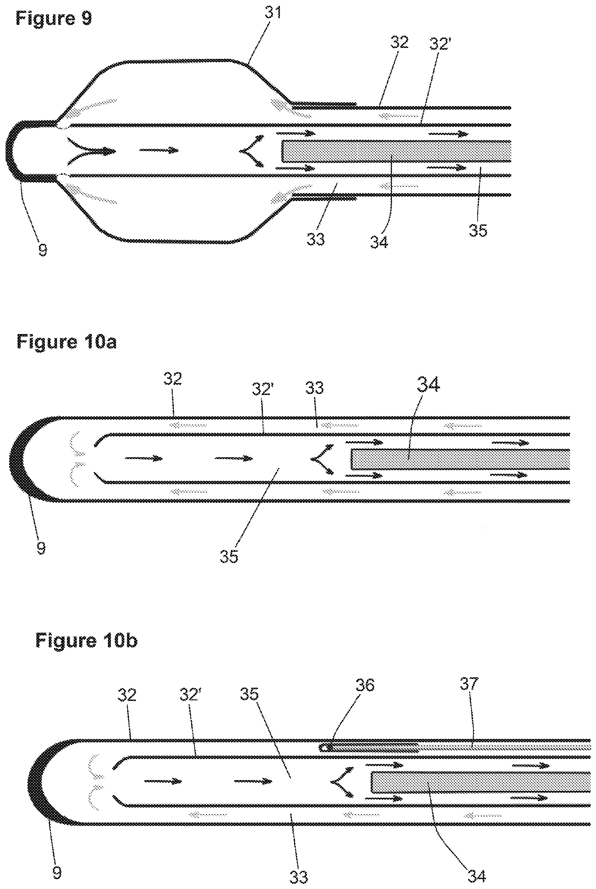

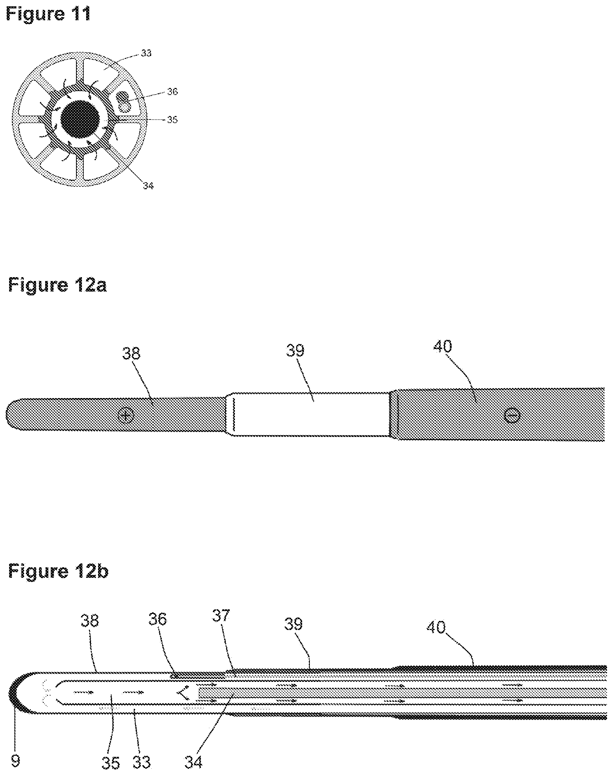

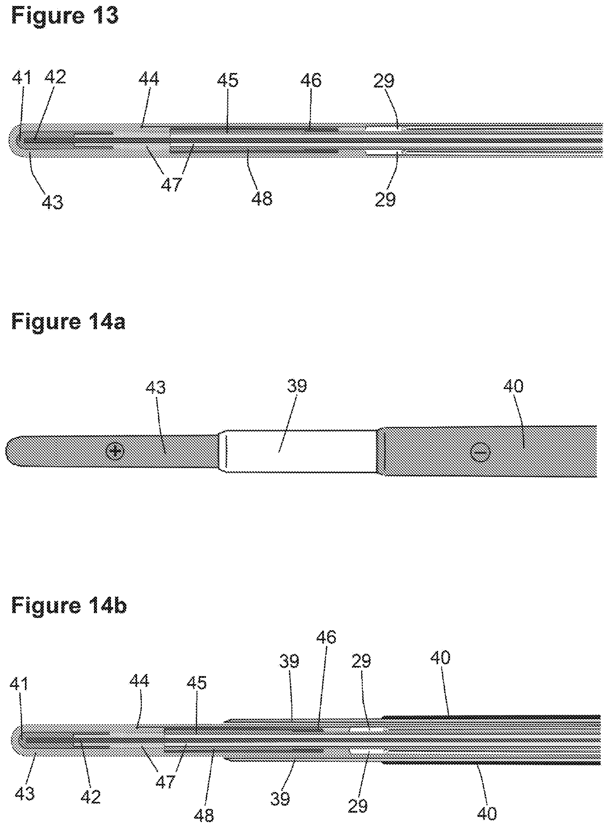

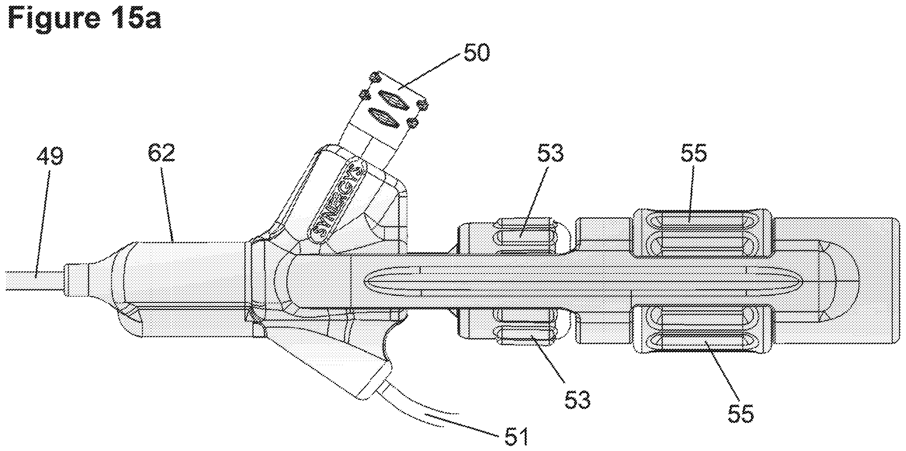

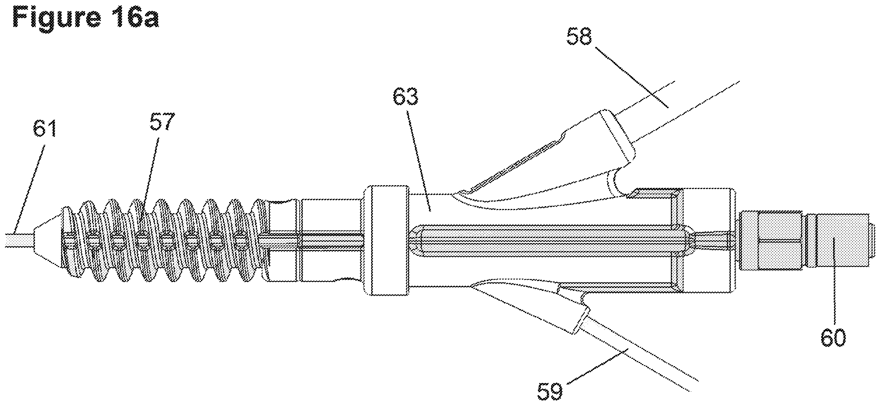

1. Example of an EM wave generator according to the invention.
2a. Bipolar RF catheter with variable energy field given by an expandable metallic stent in expanded configuration.
2b. Bipolar RF catheter with variable energy field given by a metallic stent in pre-expansion configuration.
2c. Bipolar RF catheter with variable energy field given by an expandable metallic stent collapsed into an outer catheter in closed configuration.
3a. Bipolar RF catheter based on an inner catheter acting as anode and a cathode electrode realized with two expandable and recapturable stents.
3b. Same bipolar RF catheter shown in Figure 3a in a partially closed configuration (one stent deployed and one collapsed).
3c. Same bipolar RF catheter shown in Figure 3a in a closed configuration.
4a. Bipolar RF catheter like the embodiment represented in Figure 3a. There is a single stent cathode partially deployed.
4b. Same bipolar RF catheter shown in Figure 4a in a closed configuration.
5a. to 5i. Same bipolar RF catheter device represented in Figure 4a. The ablation procedure is completed (Figure 5a), the stent left in place expanded in the conduit (Figure 5b) and the catheter retracted (Figure 5c) after detaching the electrical tethering. Re-introducing the bipolar RF catheter (Figure 5d) and eventually, if needed, collapsing, and repositioning the stent using the same electrical tethering (Figure 5e). The stent can be left in place at long-term or removed with a recapture in the same catheter (Figure 5f). A stent is positioned in a blood vessel or conduit (Figure 5g). A catheter device carrying on an ablation unit collapsed inside is shown in Figure 5h. A second stent is temporarily deployed inside the first one and an RF ablation is performed (Figure 5i).
6a. Multipolar needle/catheter for extended tissue ablations. The outer anodic catheter is carved out exposing for a predetermined length the cathodic inner catheter.
6b. Monopolar needle/catheter for extended tissue ablations. The outer anodic catheter is carved out exposing for a predetermined length the cathodic inner catheter. For this monopolar configuration is indicated the presence of a ground plate.
7a. Bipolar biopsy tweezer-ablation catheter with cooling system, integrated with a variable field ablation needle/catheter.
7b. Longitudinal section of bipolar biopsy tweezer-ablation catheter represented in Figure 7a.
8a. Monopolar biopsy tweezer-ablation catheter with cooling system as described in Figure 7a and 7b, but without the negatively charged electrode replaced by a ground plate.
8b. Longitudinal section of monopolar biopsy tweezer-ablation catheter represented in Figure 8a.
9. Laser ablation catheter (longitudinal section) with a balloon in expanded configuration acting as cooling circuit.
10a. Laser ablation catheter longitudinal section with variable energy field and associated cooling system.
10b. Laser ablation catheter longitudinal section with variable energy field and associated cooling system and thermocouple.
11. Cross section of the laser catheter showed in Figure 10b.
12a. LS ablation catheter and combined with bipolar RF ablation.
12b. Longitudinal section of the same represented in Figure 12a.
13. Longitudinal section of MW catheter for ablating tissues with insulating elements and cooling circuit.
14a. Same configuration of MW catheter of Figure 13 with the possibility of performing an RF ablation in bipolar configuration.
14b. Longitudinal section of the same represented in Figure 14a.
15a. Handle of the navigation catheter with its ports and connection cables.
15b. Longitudinal section of the same represented in Figure 15a.
16a. Handle of the active needle/catheter (transponder, RF, MW or LS) with its ports and connection cables.
16b. Longitudinal section of the same represented in Figure 16a.
17. Handle of the multisource ablation system assembled in its final configuration. It is composed by a navigation catheter and an active needle/catheter connected to its proximal end.

### Numerical references used in the figures

1. EM wave generator
2. Screen
3. RF output
4. MW output
5. LS output
6. RF & MW or RF & LS output
7. Anatomic conduit, e.g., blood vessel
8. Expandable metal stent
9. Catheter tip
10. Internal catheter shaft (anode)
11. Proximal external catheter shaft (cathode)
12. Distal external catheter shaft (cathode)
13. Inner lumen anatomic conduit
14. Irrigation interspace
15. Introducer cannula
16. First positively charged electrode (cathode)
17. Second positively charged electrode (cathode)
18. Inner negatively charged electrode (anode)
19. Outer shaft
20. Hook connector
21. Inner catheter shaft (cathode)
21'. Inner catheter shaft not electrically charged
22. Outer catheter shaft (anode)
23. Ground plate
24. Biopsy jaws (cathode)
25. Hinge mechanism
26. Inner cathodic catheter shaft
27. Insulation layer
28. Outer anodic catheter shaft
29. Refrigeration circuit
30. Outer shaft
31. Expandable balloon
32. Outer catheter shaft
32'. Inner catheter shaft
33. Cooling gas/liquid inlet lumen
34. Laser optical fiber
35. Cooling gas/liquid outlet lumen
36. Thermocouple with optical insulator
37. Wires connecting thermocouple to generator
38. Inner catheter shaft (cathode)
39. Insulating cover
40. Outer catheter shaft (anode)
41. Active steel electrode tip
42. MW antenna
43. MW catheter shaft
44. Thermocouple/thermistor
45. Conductive layer of the MW antenna
46. Balun steel tube
47. Insulating element
48. Insulating element for balun tube
49. Navigation catheter RF cannula with thermocouple
50. Connector for flushing the lumen of RF cannula
51. Connection cable
52. Sealing valve
53. Knob for closure/opening of the sealing valve
54. Female endless thread rack
55. Knob of navigation catheter
56. Internal lumen of the navigation catheter
57. Male endless thread rack
58. Cooling system
58'. Inlet lumen for cooling with fluid/gas
58". Outlet lumen for cooling with fluid/gas
59. Cable for energy delivery
60. Connection plug to generator
61. Active ablation needle/catheter
62. Handle of navigation RF catheter platform
63. Handle of active ablation needle/catheter

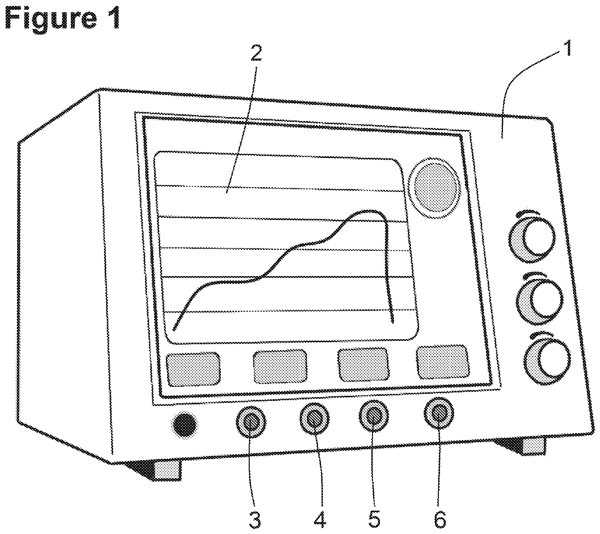

The EM wave generator **1** described in Figure 1 is equipped with a screen **2** showing the ablation parameters and typically the temperature increase ramp or the impedance and a series of EM wave outputs such as an RF output **3,** a MW output **4,** a LS **5** and joint RF and MW output **6.**

Several cursors complete the generator **1** with the function to regulate the different ablation functions.

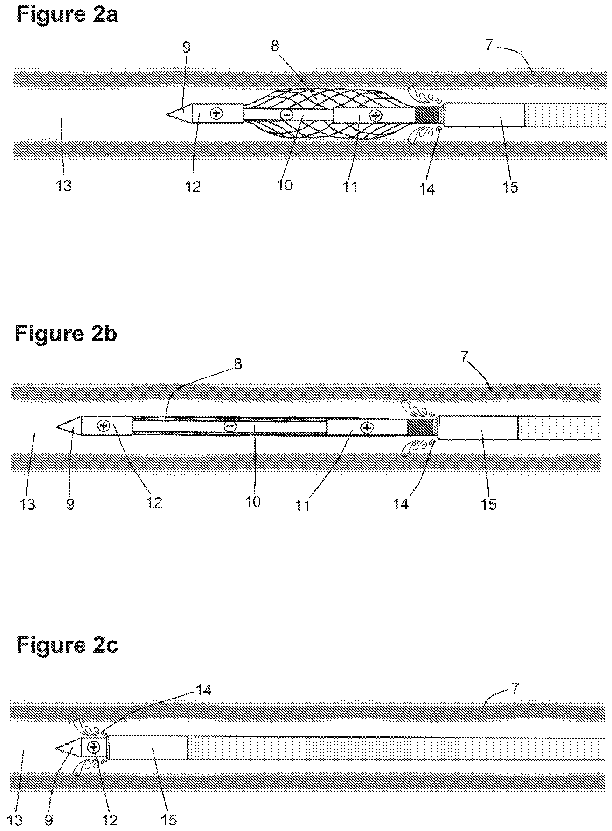

In Figure 2a an RF ablation procedure in a hollow anatomical conduit (vessel, biliary duct, etc.) is illustrated. A metallic self-expandable stent mesh **8** fits the conduit wall **7** and allows any kind of body fluids to cross it preserving a flow circulation within the conduit lumen **13.** The balloon-like self-expandable metal stent **8** is proximally anchored to a positively charged external shaft **11** and distally anchored to a positively charged external shaft **12.** The external cathode shafts **11,12** and the internal anode shaft **10** with its tip **9** are coaxial and telescopic. The external shaft has two portions: one anodic **10** and two cathodic **11,12.** The relative movement of the external shaft **11** in respect to the internal shaft **10** determines the creation of a variable electrical field able to ablate tissues of the conduit's wall **7.**

In Figures 2b and 2c the metallic stent **8** is collapsed and the external shaft **11** retracted. The distal portion of the catheter is then closed advancing the introducer cannula **15.** The system allows a flushing **14** of the interspace between the introducer cannula **15** and the internal and external shafts **10,11,12.**

In Figure 3a, 3b, 3c an example of a conduit/vessel **7** ablation associated with a stenting procedure is shown. A metallic self-expandable stent positively charged **16** is contained in a hollow negatively charged shaft **18.** A second sequential self-expandable stent **17** is collapsed in the outer flexible or rigid shaft negatively charged **19.** When the ablation unit is fully deployed (Figure 3a) a double ablation can be performed with energy fields between the stent **16** and the hollow shaft **18** and between the stent **17** and the outer shaft **19.** In Figure 3b the ablation is performed by establishing an energy field between the stent **16** and outer shaft **19.** In Figure 3c the catheter is closed and ready to be retrieved.

In another embodiment a single stent tissue ablation can be performed (Figure 4a and 4b). The stent **16,** positively charged, when deployed establish an energy field with the distal portion **18,** negatively charged, of the outer shaft **19.**

The ablation procedure can be performed and the stent left in place, at the end, to provide mechanical support to the conduit (Figure 5a to 5f). The stent **16** is firstly deployed into the conduit and kept tethered by a mechanical and electrical connection **20** to the outer catheter **18.** The stent **16** is acting as a cathode through the connection **20** while the distal part **18** of the outer shaft **19** is acting as an anode. The energy field is generated between the cathode and the anode. In Figure 5c the stent is left in place. In figure 5d the outer shaft **19** is reintroduced into the conduit, the tip **9** crosses the stent **16,** the tether **20** is exposed and connected to the stent **16** (Figure 5e). A second ablation procedure can be performed and the stent **16** thereafter can be simply retrieved (Figure 5f). This solution allows to treat tumors in anatomical conduits granting a mechanical support to preserve the conduit patency and at the same time to treat with RF tumor lesion. The advantage to have a tethering system is that the tumor ablation can be performed several times leaving the stent in place. In an additional embodiment in case of a stent restenosis in which an atherosclerotic plaque in a coronary or peripheral blood vessel or a tumor proliferation in conduit a RF ablation can be performed. In Figure 5g a stent **16** implanted in a vessel/conduit is represented and it is crossed with a catheter **19** carrying on a stent collapsed inside (Figure 5h). when the catheter **19** is positioned inside the stent **16** an active RF stent positively charged is deployed over the previous one. The shaft of the catheter **19** is then negatively charged an RF ablation of the stented portion can be realized to treat the restenosis (Figure 5i). The active RF stent can be self-expandable in Nitinol or similar alloy or can be balloon-expandable when more mechanical support is required.

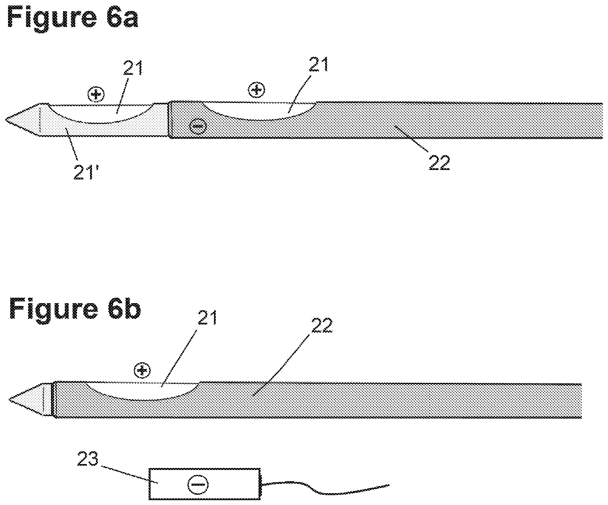

A telescopic bipolar needle/catheter emitting two ablation energy fields as described in Figure 6a. The internal catheter shaft **21** acts as cathode while the outer one **22** is acting as anode. The two catheters **21',22** are moving relatively each other creating a variable ablation field. In the present embodiment the external catheter shafts **21',22** are scalloped for a certain length exposing the surface of the internal catheter shaft **21.** This condition is creating a second ablation field. With this solution two contiguous ablation fields are generated allowing an extended ablation surface. Several alternatives are provided for this ablation catheter including the number, the length and the shape of the scalloped ablation surfaces. In the embodiment of figure 6b the needle/catheter is monopolar with a ground **23.** The outer shaft **22** has a scallop showing the inner shaft **21** acting as cathode.

In figure 7a, 7b and 8a, 8b a bipolar and monopolar telescopic ablation needles/catheters with biopsy and drug injection capability are respectively described. In the bipolar configuration (Figure 7a) the inner cathodic shaft **26,** carrying-on the biopsy jaws **24,** is inserted into the outer anodic shaft **28** insulated by an insulation layer **27.** The ensemble is contained into an outer catheter shaft **30.** In figure 7b the longitudinal section of the bipolar ablation needle/catheter is represented. The two structures **26,28** are telescopic and the relative movement can determine a different dimension of the energy ablation field. An additional embodiment of this ablation needle/catheter is the possibility to harvest biopsy samples from the ablated tissue and to inject solutions or drugs before, during and after the ablation procedure.

In Figure 8a the ablation needle/catheter is represented in monopolar configuration. The inner cathodic shaft **26** is directly contained into the outer shaft **30.** The longitudinal section of the device is shown in Figure 8b.

A LS tissue ablation application is described in Figure 9. The LS optical fiber **34** acts as internal shaft of an external multi-lumen catheter **32.** In this embodiment the LS ablation catheter can be fluid/gas cooled to mitigate the risk of tissue carbonization, a critical issue of the laser ablation procedures.

The LS fiber **34** is contained into an inner catheter shaft **32';** outside there is an outer catheter shaft **32.** A polymeric balloon **31** is obtained by sealing its proximal end on the shaft of the outer catheter shaft **32** and the distal end to the tip **9.** The balloon **31** acts as an expansion chamber for the coolant (gas preferably). In fact, when the LS ablation procedure is ongoing the optical fiber **34** can reach quite high temperatures and must be cooled down. The coolant is pumped into the interspace **33** between the outer **32** and the inner **32'** shafts. It expands into the balloon and, through holes in the inner shaft **32',** it flows back inside the interspace **35** created by the inner shaft **32'** and the optical fiber **34.** The gas-cooling system is maintained with CO₂ gas, or other gases/liquids.

In another embodiment a catheter-based LS, as above described, is realized in a way to better navigate into anatomical conduits (e.g. biliary duct or blood vessels) including an over-the-wire or a rapid exchange solution.

Alternative embodiments of LS ablation needles/catheters are described in Figures 10a, 10b, 11, 12a and 12b.

In Figures 10a and 10b two needle/catheters are described in longitudinal section. The cooling system is similar to that one described in Figure 9. The coolant is pumped through the interspace **33** and returns through the interspace **35.** The expansion chamber is at level of the tip **9.** The embodiment in Figure 10b is equipped by a thermocouple **36** located close to the distal end of the LS optical fiber **34.** This allows to obtain realistic temperature measurements of the ablated tissue. In Figure 11 the same embodiment described in Figure 10b is shown in cross-section.

In Figure 12a and 12b the above-described LS ablation needle/catheter is represented in a hybrid RF+LS configuration. It means that this device can deliver at the same time or sequentially two energies. The LS needle/catheter **38** has a metallic inner shaft portion acting as cathode and is inserted in an outer shaft **40** acting as anode separated by an insulation layer **39.** The system is telescopic therefore the relative variations in length of the shafts **38** and **40** can induce a dimensional change of the ablation energy fields generated by LS or RF.

A MW ablation needle/catheter design is described in the longitudinal section of Figure 13. This MW needle/catheter is equipped with a cooling system **29** that limits the temperature effects to the distal portion of the needle/catheter. This MW needle/catheter has an antenna **42** and a thermocouple **44.** Around the antenna conductive materials and insulating materials are stratified in different layers with a balun system **46** to impede the electrical return towards the generator increasing the electrical impedance and requiring a sharp increase of the power needed and consequently of the temperature, to complete the ablation procedure.

A hybrid RF and MW ablation needle/catheter is represented in Figure 14a and 14b (longitudinal section) and has a design like that one previously described in Figure 13. The hybrid RF+MW ablation needle/catheter is characterized by a negatively charged external shaft **40** with inside, moving telescopically, a positively charged internal shaft **43** separated by an insulation layer **39.** This ablation needle/catheter represented in this embodiment can perform an ablation procedure with only RF, only MW or in case alternatively RF and MW with a predefined and programmable time frame. This inventive solution could bypass the limitations of both ablation treatments providing an optimized ablation procedure. The MW antenna **42** is positioned inside the inner shaft **43** as well the thermocouple **44** measuring the temperature during the ablation procedure. The cooling system is provided by an inlet and outlet coolant interspace between the MW antenna conductive layer **45** and the catheter body **43** ending in an expansion room **29.** The interspace between the internal lumen **40** and the shaft **39** is suitable for injection of purging liquids or delivery drugs.

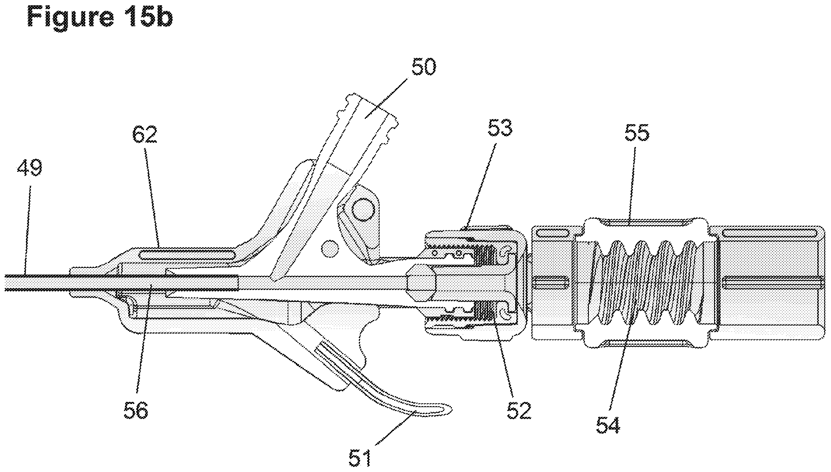

In Figure 15a and 15b (longitudinal section) the conceptual design of the navigation catheter **62** is described. The function of this navigation catheter is to serve as a hub to interchange, navigate and precisely position different active ablation needles/catheters **63** into the tumor lesions. The navigation catheter platform **62** is distally equipped with a flushing connector **50** to flush the interspace **56** between the RF cannula **49** and the active ablation needle/catheter **61.** The RF and thermocouple connections are granted by the cable **51** that connects with the generator **1.**

The sealing valve **53** placed in the mid-portion impedes the return of fluids during the ablation or flushing. On the proximal end of the navigation catheter **62** there is modular section with a knob **55** which contains a female screw endless rack **54** receiving a equal male screw endless rack **57** from the handle of the active ablation needle/catheter **63.**

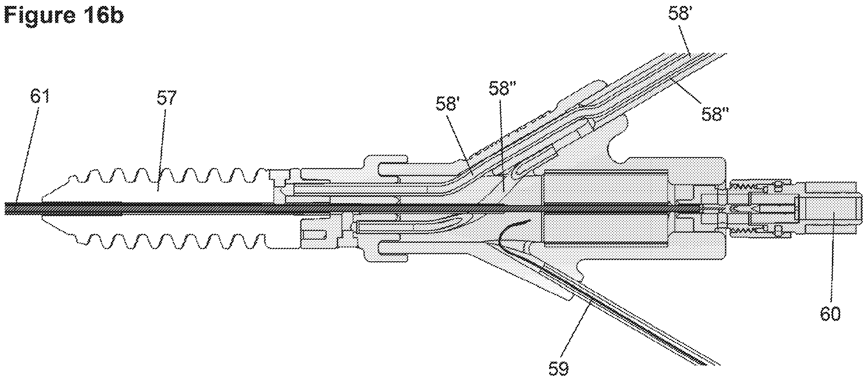

The handle of the active ablation needle/catheter **63** is described in the Figures 16a and 16b (longitudinal section). The handle of the active ablation needle/catheter **63** is conceived to mount different energy delivery solutions (single or hybrid with combination of RF+MW or RF+LS). In an embodiment consisting in a hybrid solution the proximal portion of the active ablation needle/catheter handle **63** a connector for inlet and outlet cooling systems **58',58"** is present as well as the cable **59** for the thermocouple connection to the generator **1.** The different energy delivery to the active ablation needle/catheter **61** is placed on its proximal end where a connector **60** is placed to connect a cable to the generator **1.**

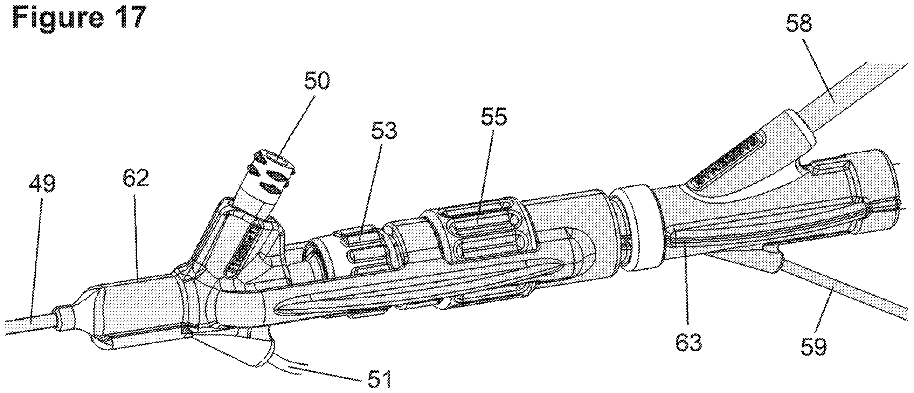

The complete ablation system is described in Figure 17. The two components: the handle of the navigation catheter **62** and the handle carrying on the active ablation needle/catheter **63** are assembled.

The invention is of course not limited to the above cited examples.

## Claims

1. Tissue ablation system for the internal treatment of parenchymal organs, hollow anatomical conduits or blood vessels (7) ; said system comprising an electromagnetic, EM, wave generator (1) and a catheter (8-14,16-40) with an active distal end; **characterized by** the fact that said generator (1) includes at least two EM wave outputs (3-6) and is adapted to provide three types of EM waves through said outputs (3-6), namely radiofrequency, RF, microwave, MW and laser, LS; said generator (1) furthermore comprising a processing unit that is programmed, among other things, to emit all three EM waves at the same time and control the interaction among them.

2. System according to claim 1 wherein one output (6) is a joint output that provides two types of EM waves.

3. System according to claim 1 or 2 comprising four outputs (3-6) .

4. System according to any of claims 1 to 3 wherein said catheter comprises an internal shaft (10,21,21',26,32',34,38) and an external hollow shaft (11,12,19,22,32,40), both shafts being coaxial and movable relatively to each other.

5. System according to claim 4 wherein said catheter is a laser-based catheter, wherein the internal shaft is an optic fiber (34) with a free portion and wherein the amplitude of the laser ablation field dimension is telescopically regulated by the relative movement of the external shaft (32).

6. System according to claim 5 wherein said laser-based catheter furthermore comprises coolant outlets (35) that are adapted to provide a coolant around the free portion of the optical fiber (34).

7. System according to claim 6 comprising an expandable balloon (31) fixed to the distal end of the external shaft (32), said balloon (31) acting as an expansion chamber for the coolant.

8. System according to claim 4 wherein said catheter comprises an anodic portion and a cathodic portion.

9. System according to claim 8 wherein said anodic portion is located on the internal shaft (10) and wherein said cathodic portion is located on two external shafts (11,12).

10. System according to claim 9 comprising a balloon-like metallic mesh (8) being located around the catheter distal end in such a way as to conductively connect the two external shafts (11,12).

11. System according to claim 9 comprising biopsy jaws (24) that are linked to the internal shaft (26) distal end.

12. System according to claim 9 comprising an antenna (42) located within the internal shaft (35) and wherein said catheter is adapted to provide MW alone, RF alone or a combination of both.

13. System according to anyone of the previous claims comprising a handle (62) for a navigation catheter and a handle (63) for the active catheter (8-14,16-40) or a needle.

## Patentansprüche

1. Gewebeablationssystem zur inneren Behandlung von parenchymalen Organen, hohlen anatomischen Leitungen oder Blutgefäßen (7); wobei das System einen Generator (1) für elektromagnetische, EM, Wellen und einen Katheter (8-14, 16-40) mit einem aktiven distalen Ende umfasst; **gekennzeichnet durch** die Tatsache, dass der Generator (1) mindestens zwei EM-Wellenausgänge (3-6) einschließt und eingerichtet ist, um durch diese Ausgänge (3-6) drei Typen von EM-Wellen bereitzustellen, nämlich Hochfrequenz, HF, Mikrowelle, MW, und Laser, LS; wobei der Generator (1) zudem eine Verarbeitungseinheit umfasst, die unter anderem programmiert ist, um alle drei EM-Wellen zur gleichen Zeit zu emittieren und die Interaktion zwischen ihnen zu steuern.

2. System nach Anspruch 1, wobei ein Ausgang (6) ein gemeinsamer Ausgang ist, der zwei Typen von EM-Wellen bereitstellt.

3. System nach Anspruch 1 oder 2, umfassend vier Ausgänge (3-6).

4. System nach einem der Ansprüche 1 bis 3, wobei der Katheter einen inneren Schaft (10, 21, 21', 26, 32', 34, 38) und einen äußeren hohlen Schaft (11, 12, 19, 22, 32, 40) umfasst, wobei beide Schäfte koaxial und relativ zueinander beweglich sind.

5. System nach Anspruch 4, wobei der Katheter ein Katheter auf Laserbasis ist, wobei der innere Schaft eine Lichtleiterfaser (34) mit einem freien Abschnitt ist, und wobei die Amplitude der Laserablationsfeldabmessung teleskopartig durch die relative Bewegung des äußeren Schafts (32) geregelt wird.

6. System nach Anspruch 5, wobei der Katheter auf Laserbasis des Weiteren Kühlmittelausgänge (35) umfasst, die eingerichtet sind, um ein Kühlmittel um den freien Abschnitt der Lichtleiterfaser (34) herum bereitzustellen.

7. System nach Anspruch 6, umfassend einen expandierbaren Ballon (31), der an dem distalen Ende des äußeren Schafts (32) fixiert ist, wobei der Ballon (31) als Expansionskammer für das Kühlmittel wirkt.

8. System nach Anspruch 4, wobei der Katheter einen anodischen Abschnitt und einen kathodischen Abschnitt umfasst.

9. System nach Anspruch 8, wobei der anodische Abschnitt sich auf dem inneren Schaft (10) befindet, und wobei der kathodische Abschnitt sich auf zwei äußeren Schäften (11, 12) befindet.

10. System nach Anspruch 9, umfassend ein ballonartiges metallisches Maschenmaterial (8), das sich in einer solchen Weise um das distale Ende des Katheters herum befindet, dass die beiden äußeren Schäfte (11, 12) leitfähig verbunden sind.

11. System nach Anspruch 9, umfassend Biopsieklemmbacken (24), die mit dem distalen Ende des inneren Schafts (26) verbunden sind.

12. System nach Anspruch 9, umfassend eine Antenne (42), die sich innerhalb des inneren Schafts (35) befindet, und wobei der Katheter eingerichtet ist, um nur MW, nur HF oder eine Kombination von beiden bereitzustellen.

13. System nach einem der vorhergehenden Ansprüche, umfassend einen Griff (62) für einen Navigationskatheter und einen Griff (63) für den aktiven Katheter (8-14, 16-40) oder eine Nadel.

## Revendications

1. Système d'ablation de tissu pour le traitement interne d'organes parenchymateux, de conduits anatomiques creux ou de vaisseaux sanguins (7) ; ledit système comprenant un générateur d'ondes électromagnétiques, EM, (1) et un cathéter (8-14, 16-40) avec une extrémité distale active ; **caractérisé en ce que** ledit générateur (1) comprend au moins deux sorties d'ondes EM (3-6) et est adapté pour fournir trois types d'ondes EM au moyen desdites sorties (3-6), à savoir radiofréquence, RF, micro-ondes, MW et laser, LS ; ledit générateur (1) comprenant en outre une unité de traitement qui est programmée, entre autres, pour émettre les trois ondes EM en même temps et commander l'interaction entre elles.

2. Système selon la revendication 1, une sortie (6) étant une sortie commune qui fournit deux types d'ondes EM.

3. Système selon la revendication 1 ou 2, comprenant quatre sorties (3-6).

4. Système selon l'une quelconque des revendications 1 à 3, ledit cathéter comprenant un arbre interne (10, 21, 21', 26, 32', 34, 38) et un arbre creux externe (11, 12, 19, 22, 32, 40), les deux arbres étant coaxiaux et mobiles relativement l'un par rapport à l'autre.

5. Système selon la revendication 4, ledit cathéter étant un cathéter à base de laser, l'arbre interne étant une fibre optique (34) avec une partie libre et l'amplitude de la dimension du champ d'ablation laser étant régulée de manière télescopique par le mouvement relatif de l'arbre externe (32).

6. Système selon la revendication 5, ledit cathéter à base de laser comprenant en outre des sorties de liquide de refroidissement (35) qui sont adaptées pour fournir un liquide de refroidissement autour de la partie libre de la fibre optique (34).

7. Système selon la revendication 6, comprenant un ballonnet extensible (31) fixé à l'extrémité distale de l'arbre externe (32), ledit ballonnet (31) faisant office de chambre d'expansion pour le liquide de refroidissement.

8. Système selon la revendication 4, ledit cathéter comprenant une partie anodique et une partie cathodique.

9. Système selon la revendication 8, ladite partie anodique étant située sur l'arbre interne (10) et ladite partie cathodique étant située sur deux arbres externes (11, 12).

10. Système selon la revendication 9, comprenant un maillage métallique en forme de ballonnet (8) qui est situé autour de l'extrémité distale du cathéter de manière à relier par conduction les deux arbres externes (11, 12).

11. Système selon la revendication 9, comprenant des mâchoires de biopsie (24) qui sont reliées à l'extrémité distale de l'arbre interne (26).

12. Système selon la revendication 9, comprenant une antenne (42) située à l'intérieur de l'arbre interne (35) et ledit cathéter étant adapté pour fournir des MW seuls, des RF seuls ou une combinaison des deux.

13. Système selon l'une quelconque des revendications précédentes, comprenant une poignée (62) pour un cathéter de navigation et une poignée (63) pour le cathéter actif (8-14, 16-40) ou une aiguille.
